# EUROPEAN PATENT APPLICATION

(11) **EP 1 624 054 A1**
(43) Date of publication of application: **08.02.2006**
(21) Application number: 04731488.5
(22) Date of filing: 06.05.2004
(51) Int. Cl.: C12N 5/06, C12N 5/08

(54) **CELL CULTURE METHOD AND CULTURED TISSUE**

(30) Priority: 14.05.2003 JP 2003136336
(71) Applicant: Japan Tissue Engineering Co., Ltd., Gamagori-shi, Aichi 443-0022 (JP); Honda, Hiroyuki, Nagoya-shi, Aichi 458-0805 (JP)
(72) Inventor: HONDA, Hiroyuki, Nagoya-shi, Aichi 4580805 (JP); ITO, Akira, Nagoya-shi, Aichi 458-0827 (JP); KOBAYASHI, Takeshi, Kasugai-shi, Aichi 487-0013 (JP); UEDA, Minoru, Nagoya-shi, Aichi 4610011 (JP); KAGAMI, Hideaki, Sunpark Shigahondori, Nagoya-shi, Aichi 462-0831 (JP); HATA, Ken-ichiro, Kariya-shi, Aichi 448-0802 (JP)
(74) Representative: Kuhnen & Wacker
(86) International application number: PCT/JP2004/006409
(87) International publication number: WO 2004/101774

(57) **Abstract**

A cell culture method prepares first cells that are adhesion-dependent cells and monolayered or multilayered cells on a culture surface of a culture substrate, seeds second cells that are adhesion-dependent cells and are magnetized by allowing to have magnetic particles on the first cells, induces the second cells to a predetermined position on the first cells by magnetic force, and cultures the first cells and the second cells in a cell arrangement obtained by the magnetic induction. According to this cell culture method, after a cell sheet was prepared individually, cells can be multilayered without changing a temperature, peeling the monolayered sheet and laminating the monolayered sheets.

## Description

### TECHNICAL FIELD

The present invention relates to a cell culture method and cultured tissue.

### BACKGROUND ART

Recently, in accordance with the development of tissue engineering, many cultured tissues have been reconstructed in vitro. For example, cultured tissue having a relatively simple tissue structure that is two-dimensionally structured by single cells such as epidermal cells, and cultured tissue such as cultured cartridge, cultured epidermis, etc. having a three-dimensional tissue structure in which a scaffold such as gel, sponge, or the like, is allowed to hold chondrocytes or fibroblasts have been known.

However, there remain many problems in constructing organs having a complicated structure and functions, that is, heart, liver, kidney, brain, and the like, by technologies of tissue engineering, and various studies are increasingly being performed. The blood vessels and organs are composed of various types of cells. In order to construct them in vitro, it is necessary to position various types of cells to respective suitable positions. However, it has been indicated that in an in vitro environment, different types of cells do not adhere to each other, and disclosed that sufficient functions are not expressed ("Journal of Biomedical Materials Research" 1999; 45: p.355-362 (U.S.)). On the other hand, there has been.proposed a method using a culture container on which a temperature responsive polymer is laid. This method constructs three-dimensional cultured tissue by culturing cells at high density to form a cell sheet; then recovering the cell sheet by changing environmental temperature without enzyme treatment; and laminating cell sheets of different types of cells ("Journal of Biomedical Materials Research" 2002; 62: p. 464-470 (U.S.)).

### DISCLOSURE OF THE INVENTION

The formation of multilayered cell sheet by using the temperature responsive polymer mentioned above needs procedures of preparing a monolayered sheet in a culture container, then separating the sheet from the culture container by changing a temperature, and laminating the monolayered sheets. The procedure is very complicated and poor in workability as a method of constructing various three-dimensional cultured tissues. It takes much time and labor to recover a cell sheet by changing a temperature and it is difficult to handle a monolayer cell sheet. In addition, in constructing three-dimensional cultured tissue, not only adhesion of cells but also positioning thereof are important.

The present invention was made with the foregoing problems in mind, and an object of the present invention is to provide a cell culture method capable of obtaining a multilayered cell sheet without preparing cell sheets individually, separating the cell sheets indivisually from a culture container, or laminating the cell sheets. Another object of the present invention is to provide a cell culture method capable of multilayering cells without considerably changing a temperature. A further object of the present invention is to provide a cell culture method capable of constructing three-dimensional cultured tissue in a simple way. A yet further object of the present invention is to provide novel cultured tissue.

In order to achieve at least one of the above-mentioned objects, the present invention employs the following technique. The present invention provides a cell culture method including: a preparation step that prepares first cells on a culture surface of a culture substrate, the first cells being adhesion-dependent cells and monolayered or multilayered cells; a seeding step that seeds second cells on the first cells, the second cells being adhesion-dependent cells and magnetized cells; a magnetic induction step that attracts the second cells to a predetermined position on the first cells by magnetic force; and a culture step that cultures the first cells and the second cells in a cell arrangement obtained by the magnetic induction step. Furthermore, the present invention also provides a cell culture method, including a preparation step that prepares first cells on a culture surface of a culture substrate, the first cells being adhesion-dependent cells and monolayered or multilayered cells; a seeding step that seeds second cells on the first cells, the second cells being adhesion-dependent cells and magnetized cells; and a magnetic induction step that attracts the second cells to a predetermined position on the first cells by magnetic force.

In any of these cell culture methods, it is preferable that the second cells are seeded in a state in which they are monolayered or multilayered. It is also preferable that the preparation step includes a culture step that cultures the first cells until a monolayered or multilayered cell sheet is formed. Furthermore, it is preferable that the preparation step includes a magnetic induction step that attracts magnetized first cells to a culture surface of a culture substrate by magnetic force. It is also preferable that the culture substrate for culturing the first cells and the second cells has any of a plate shape, a tubular shape, an envelope shape having a hollow part, a columnar shape, a dish shape and a spherical shape. Furthermore, it is preferable that the culture surface of the culture substrate is a cell non-adhesive surface. Furthermore, a combination of the first cells and the second cells may be a combination of different types of cells. In particular, the combination of the first cells and the second cells may be any of combinations of parencymal hepatocytes and vascular endothelial cells, fibroblasts and epithelial cells, smooth muscle cells and vascular endothelial cells, keratocytes and corneal epithelial cells, and keratocytes and corneal endothelial cells.

These cell culture methods may include a releasing step that release cultured cells obtained in the culture step from the culture substrate, and may also include a recovering step of the cultured cells. Furthermore, the present invention provides cultured tissue having a multilayered product of cells, wherein at least a part of the multilayered product of cells includes magnetized cells.

### BRIEF DESCRIPTION OF THE DRAWINGS

Fig. 1 shows one example of a magnetic particle cationic liposome (MPCL).
Fig. 2 shows one example of an antibody-immobilized magnetoliposome (AML).
Fig. 3 shows one example of a cell culture method of the present invention.
Fig. 4 shows another example of a cell culture method of the present invention.
Fig. 5 shows various forms of three-dimensional cultured tissues obtained by the culture method of the present invention. Fig. 5(a) shows sheet-like cultured tissue including a single type of cells that are multilayered; Fig. 5(b) shows a sheet-like cultured tissue including different types of cells that are multilayered; Fig. 5(c) shows a tubular-shaped cultured tissue including different types of cells that are multilayered; and Fig. 5(d) shows a circular dish-shaped cultured tissue including different types of cells that are multilayered.
Fig. 6 schematically shows Example 1, showing a process for constructing cultured liver tissue, which co-cultures parencymal hepatocytes and magnetized non-parencymal hepatocytes (human vascular endothelial cells) with magnetic force applied.
Fig. 7 is a graph showing the measurement results of intracellular iron concentration of HAECs.
Fig. 8 is a graph showing the measurement results of number of viable cells of HAECs.
Fig.9 is a graph showing change over time of the amount of albumin secretion in various culture systems.
Fig. 10 schematically shows Example 2.

### BEST MODE FOR CARRYING OUT THE INVENTION

Hereinafter, best modes for carrying out the present invention will be described. A cell culture method of the present invention will be described, and further cultured tissue will be described. The cell culture method of the present invention is a method that multilayers at least first cells and second cells. The cell culture method may also include seeding and culturing third cells or any more cells, and laminating (multilayering) third cell sheet or any more cell sheet. The third cells or any more cells are also cultured in accordance with the culture method of the second cells. Therefore, the following description will also apply to culturing the third cells or any more cells to form tissue.

According to a cell culture method of one embodiment of the present invention, the procedure of the method seeds magnetized second cells on monolayered or multilayered first cells, and produces magnetic induction so as to attract the second cells to a predetermined position on the first cells by magnetic force. Thereafter, the first cells and second cells can be cultured in this state. In other words, the procedure of the method positions the second cells with respect to the first cells by magnetic force and aligns the second cells on a predetermined position to form a multilayered structure. By culturing a group of multilayered cells, these cells adhere to each other. Therefore, this cell culture method can obtain multilayered cells without preparing cell sheets individually, separating the zell sheets indivisually from a container, or laminating the cell sheets. Furthermore, cells can be multilayered without considerably changing a temperature. By a magnetic induction for attracting cells to a predetermined position by magnetic force, the second cells can be positioned on a desirable position, so that a desirable multilayer structure can be constructed. Consequently, it is possible to construct three-dimensional cultured tissue in a simple way. In this cultured tissue thus constructed, desirable three-dimensional cultured tissue can be constructed and held easily by magnetic force. Furthermore, the cultured tissue can be transferred or fixed by magnetic force.

In the cell culture method of the present invention, cells to be cultured are adhesion-dependent cells. The adhesion-dependent cells adhere to a culture surface directly or indirectly, expand the adhesion area and then divide, and may be also referred to as anchorage-dependent cells. Examples of the adhesion-dependent cells may include various cells harvested from warm-blooded animals such as human, mouse, rat, guinea pig, hamster, chicken, rabbit, pig, sheep, cow, horse, dog, cat, monkey, etc. Examples of the cell of such warm-blooded animals may include keratinocyte, splenocyte, neurocyte, glia cell, pancreatic β cell, mesangium cell, Langerhans cell, epidermal cell, epithelial cell (including corneal epithelial cell, oral mucosal epithelial cell, amniotic membrane epithelial cell, etc.), endothelial cell (including vascular endothelial cell, corneal endothelial cell, etc.), fibroblast, parenchymal cell (including parencymal hepatocyte, keratocyte, etc.), muscle cell including smooth muscle cell such as vascular smooth muscle cell, adipocyte, synoviocyte, chondrocyte, osteocyte, osteoblast, osteoclast, mammary glandular cell, hepatocyte, cell derived from periosteum, or interstitial cell, or precursor cell thereof, and further stem cell such as embryonic stem cell (ESC), mesenchymal stem cell (MSC), etc., and adhesion-dependent cancer cell.

In this culture method, an organism (allogenic, xenogenic, and same individual) from which cells to be multilayered are derived is not particularly limited. Even xenogenic organisms can be co-cultured at a cell level, and thus obtained cultured tissue can be used in some applications. Note here that allogenic and xenogenic herein denote allogenic and xenogenic based on origins of cells (for example, donor and recipient at the time of transplantation, etc.), respectively. These cells may be the same type of cells or different types of cells. The same type of cells or different types of cells do not mean allogenic or xenogenic described above. As mentioned above, in the present invention, for example, a combination of the first cells and second cells can include combinations of same type of cells from allogenic animals, different types of cells from allogenic animals, same type of cells from xenogenic animals, different types of cells from xenogenic animals, same type of cells from the same individual, and different types of cells from the same individual.

According to the present invention, even different types of cells can favorably adhere to each other by magnetic force. The use of the same types of cells is preferable to construct tissue such as cardiac muscle tissue or dermal tissue (epithelial layers) greatly expressing their functions by multilayering single cells and is particularly effective in types of cells that are not easily multilayered in a general culture operation. The use of different types of cells is preferable to construct organs such as liver, kidney, blood vessel, or the like, which can achieve their functions by the mutual actions of plural types of cells. By using different types of cells, it is also possible to construct cardiac muscle tissue or dermal tissue as an organ.

An example of the use of different types of cells includes the use of a combination of parencymal hepatocytes as first cells and vascular endothelial cells as second cells. This combination is preferable for liver tissue. Other examples include a combination of fibroblasts as first cells and epithelial cells as second cells, which is preferable for dermal tissue, and a combination of smooth muscle cells as first cells and vascular endothelial cells as second cells, which is preferable for tubular-shaped vascular tissue (this is an order in the case where the inner surface of a tubular structure is made to be a culture surface). Note here that vascular tissue may be formed of vascular endothelial cells as first cells, smooth muscle cells as second cells and fibroblasts as third cells (this is an order in the case where the outer surface of a tubular structure is made to be a culture surface). The combination of different types of cells may include keratocytes as first cells and corneal epithelial cells or corneal endothelial cells as second cells and this combination is preferable for corneal tissue. In this case, when corneal epithelial cells or corneal endothelial cells are selected as third cells, corneal epithelial cells, keratocytes and corneal endothelial cells can be multilayered in this order and this combination is further preferable for corneal tissue. In the multilayer structure of different types of cells including these specific combinations, the order of the first cells, second cells, third cells, and any more cells is not particularly limited, but it may be appropriately changed dependent upon tissues or sites or forms of culture substrates. It is preferable to use a tubular structure (including a columnar structure) in the case of constructing the tubular-shaped vascular tissue, and it is preferable to use a dish-shaped structure, in particular, a circular dish-shaped structure in the case of constructing the corneal tissue.

### (Preparation step)

In a preparation step of the present invention, monolayered or multilayered first cells are prepared on a culture surface of a culture substrate. The monolayer and multilayer includes a monolayered sheet and a multilayered sheet. However, it is not always necessary that the first cells are cultured until a monolayered sheet or a multilayered sheet is formed. The first cells may not form a cell sheet, but may form monolayered or multilayered cell accumulation in which cells are attracted by magnetic force so as to be accumulated on a predetermined site of the culture surface. For example, as mentioned below, the magnetized first cells in a form of dispersion (cell suspension) may be attracted onto the culture surface by magnetic force, and thereby substantial monolayered or multilayered cells may be prepared on the culture surface. The shapes of the cell accumulation and the cell sheet are not particularly limited, but they may be formed in any shapes corresponding to the shapes of the culture surface of the culture substrate on which the first cells are accumulated or cultured.

The first cells may be held, accumulated or cultured by the use of a culture substrate having a culture surface on which cells are attached and cultured. The culture substrate defines the shape of a first cell layer that serves as a base layer of a cell product to be multilayered. However, the planar shape of the first cell layer is finally determined by a region on which magnetic force acts. The cultured tissue obtained by the method of the present invention is finally separated from the culture substrate in accordance with the purposes. Various shapes of culture substrates can be used. For constructing three-dimensional cultured tissue, it is preferable to use a culture substrate having a plate shape, tubular shape, columnar shape, envelope shape having a hollow, dish shape and spherical shape. The plate-shaped culture substrate is suitable for forming a multilayered cell sheet having various kinds of planer shapes. When the tubular-shaped culture substrate is used, by using the inner surface and/or outer surface thereof as a culture surface, tubular-shaped cultured tissue can be obtained. Furthermore, tubular-shaped cultured tissue can be also obtained by using the outer surface of a columnar-shaped culture substrate. When a columnar culture substrate is used, a columnar culture substrate which itself is a magnet can be used. Furthermore, when a culture substrate has an envelope shape having a hollow, by using the inner surface and/or outer surface thereof as a culture surface, an envelope-shaped (bag-shaped) cultured tissue can be obtained. When a tubular-shaped substrate is used as a culture substrate, in the first preparation step, a tubular-shaped cell accumulation or a cell sheet corresponding to the outer surface or inner surface of the tubular-shaped culture substrate can be obtained. When the envelope shaped culture substrate is used, an envelope-shaped cell accumulation or sheet can be obtained. When a dish-shaped culture substrate is used, by using the concave surface and/or convex surface of a culture substrate as a culture surface, dish-shaped cultured tissue can be obtained. On the other hand, when a spherical culture substrate is used, by making the sphere itself or the center of the sphere be a magnet, the first cells and the second cells can be attracted to the spherical surface and cytokine or protein can be efficiently extracted by a microcarrier culture method. In this case, cells are not separated from the culture substrate and cells are cultured together with the cell substrate.

It is preferable that the culture surface of the culture substrate used in the present invention is a cell non-adhesive surface. Herein, the "cell non-adhesive culture surface" may be any culture surfaces to which adhesion-dependent cells do not adhere or do not easily adhere. Examples of such culture surfaces may include a bottom (which may also include membrane) of a culture container made of such materials as polystyrene, polypropylene, fluororesin, polytetrafluoroethylene (PTFE), polycarbonate, polyester, etc., a culture surface (which may also include membrane) of a culture substrate coated with agarose, agar, gelatin, collagen, fibrin, etc., a positively charged culture surface of a culture substrate, and the like. Examples of the culture substrate having a cell non-adhesive culture surface may include, for example, an ultra-low-attachment plate (trade name of Corning), Hydro cell (trade name of Cell Seed), and the like. The culture surface to which cells do not easily adhere means a culture surface having adhesiveness to such an extent that when magnetic force is removed, magnetized cells can be separated from the culture surface by lightly shaking the culture substrate.

When a culture substrate having a cell non-adhesive culture surface is used, by producing magnetic induction so that the first cells are magnetized and attracted to the culture surface by magnetic force, the first cells can be cultured in a state in which they are attached to the culture surface of the culture substrate. At the same time, when cultured tissue is finally separated from the culture substrate, only by reducing or removing the magnetic force, the cultured tissue can be released and separated from the culture surface. Consequently, cultured tissue can be recovered without using an enzyme, a temperature responsive polymer, or the like. As a result, cultured tissue can be recovered without changing a temperature and later handling becomes easy.

### (Seeding step)

The seeding step of the present invention supplies the first cells with the magnetized second cells. For magnetizing cells, for example, a method for allowing cells to hold magnetized particles can be employed. Such a magnetization step may be, for example, a step of bringing magnetic particles into contact with cells for a predetermined time. Magnetic particles for magnetizing the second cells are not particularly limited, but any particles may be employed as long as they can be held by the cells and thereby provide the cells with magnetic property. Examples of such magnetic particles may include magnetic particles constituting a magnetic particle cationic liposome (MPCL) in which magnetic particles such as magnetite are enclosed in a liposome, an antibody-immobilized magnetoliposome (AML) in which magnetic particles are enclosed in an antibody-immobilized liposome; magnetic micro-beads in MACS (Magnetic Cell Sorting and Separation of Biomolecules) produced by Daiichi Pure Chemicals; magnetic nanoparticles (trade name: EasySep) produced by VERITAS, and the like. Among these magnetic particles, magnetic particles containing liposomes such as MPCL and AML are preferable because they are taken up by cells by the presence of liposomes, a single cell can take up a large number of magnetic particles, and thereby the cells can easily have a magnetic property to such an extent that the second cells can be attracted in the direction of the culture surface of the culture substrate (in the direction of the first cells) by magnetic force.

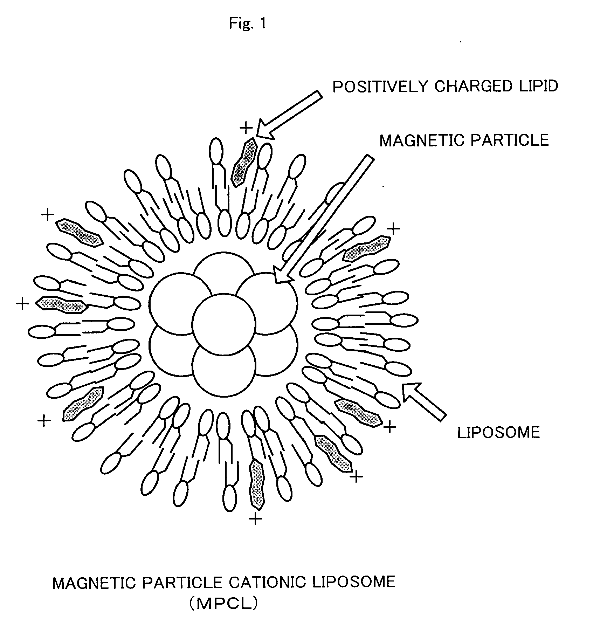

As shown in Fig. 1 as one example, the MPCL has a structure in which magnetic particles such as magnetite are enclosed in a liposome and the liposome is provided with positively charged lipid. Since many cells are negatively charged, they are easily bound to positively charged MPCLs. Since MPCLs have liposomes, they are easily taken up by cells. Consequently, the present invention, in which MPCLs are employed as the magnetic particle, can be applied for culturing various cells. MPCLs may be prepared with reference to the method for preparing magnetite cationic liposome (MCL) described in, for example, Jpn. J. Cancer Res. Vol. 87 (1996), p. 1179-1183. When MPCLs are employed in the magnetization step of cells, it is preferable that 1-150 pg/cell, particularly 20-150 pg/cell of MPCLs are used as the magnetic particles. Furthermore, in the magnetization step, it is preferable that a next step is carried out at 0.5-8 hours, particularly 3-5 hours after cells to be cultured and MPCLs start to come into contact with each other.

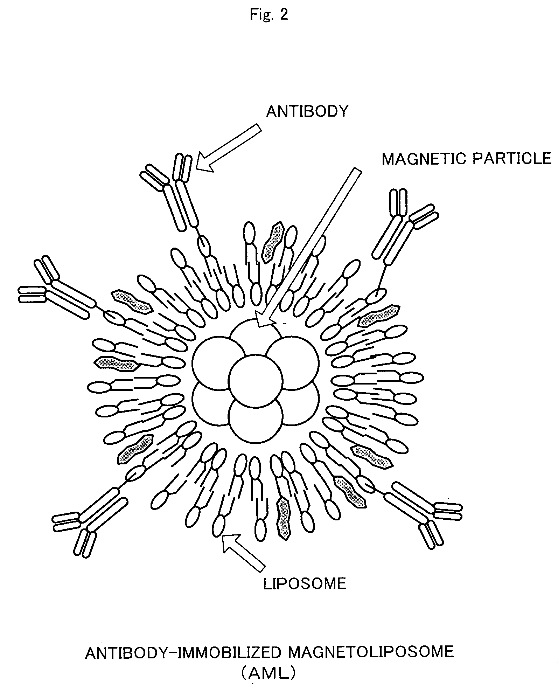

As shown in Fig. 2 as one example, AML has a structure in which magnetic particles such as magnetite are enclosed in a liposome and the liposome is provided with antibodies. As the antibody, an antibody with a property of specifically binding to certain cells to be cultured is selected. Cells having a site specifically binding to the antibody are easily bound to the antibody in the AML, and AMLs are easily taken up by cells because they have liposomes. AMLs may be prepared with reference to the preparing method described in, for example, J. Chem. Eng. Jpn. Vol. 34 (2001), p. 66-72. When AMLs are employed in the magnetization step, it is preferable that 1-150 pg/cell, particularly 20-150 pg/cell of AMLs are used as the magnetic particles. Furthermore, in the magnetization step, it is preferable that a next step is carried out at 0.5-8 hours, particularly 3-5 hours after cells to be cultured and AMLs start to come into contact with each other.

In the seeding step of the present invention, the seeding density may be appropriately set dependent upon the kind of cells, the size of targeted cultured tissue, and the like, but is generally set in the range from 1×10³ cells/cm² to 1×10⁶ cells/cm².

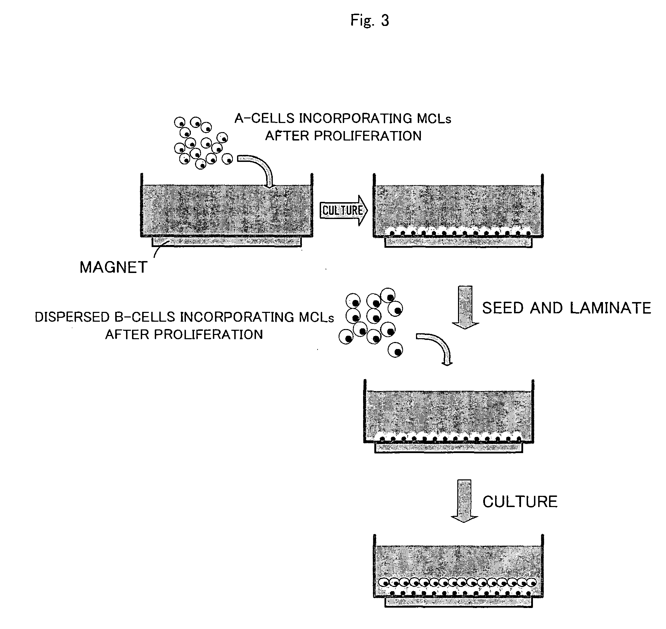

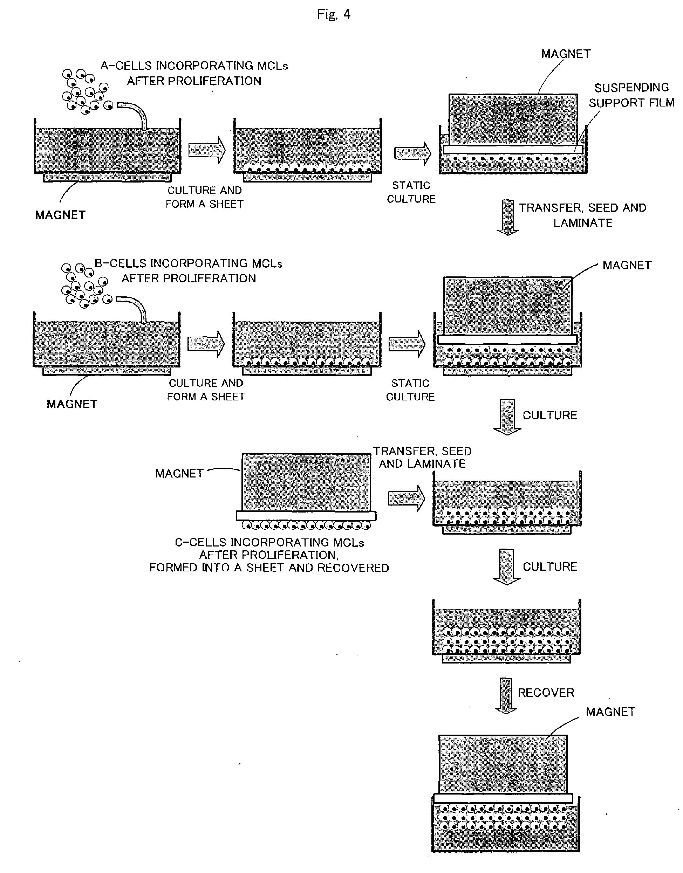

The second cells to be seeded have any forms as long as they are magnetized. The form of the second cells at the time of seeding may be in a dispersion form as shown in Fig. 3 and may be in a monolayered or multilayered state as shown in Fig. 4. Furthermore, they may be in a form of a monolayered cell sheet or a multilayered cell sheet. The magnetized cells in a dispersion form can be obtained by bringing the above-mentioned magnetic particles into contact with the second cells for a predetermined time. Monolayered or multilayered magnetized cells (respectively including a sheet product, the same is true hereafter) can be obtained by seeding temporarily magnetized second cells on a culture surface of an appropriate culture substrate, and culturing them while producing magnetic induction so as to attract the magnetized cells to the culture surface by magnetic force. Furthermore, the monolayered or multilayered magnetized cells can be also obtained easily by culturing the second cells in a form of a monolayer or multilayer structure, and then bringing them into contact with magnetic particles. The structure of the second cells may be selected between monolayered or multilayered structure as necessary, and appropriate culture conditions such as medium compositions may be employed in accordance with the structure. The form of the monolayered sheet or multilayered sheet of second cells may not be necessary to correspond to the three-dimensional shape of the culture surface of the culture substrate on which the first cells are prepared. Since the cell sheet, whether monolayer cell sheet or multilayer cell sheet, has sufficient flexibility and is attracted to the culture surface by magnetic force, when it has general plane sheet shape, it can sufficiently follow the culture surface even if the culture surface has a curve. Furthermore, two or more monolayered cell sheets or multilayered cell sheets of the second cells may be supplied sequentially so as to form a multilayered structure, or previously multilayered sheet may be supplied.

A method for supplying the first cells with the magnetized second cells is not particularly limited. In case where the cells are in a dispersion form, they can be treated the same as a usual cell suspension liquid. As shown in Fig. 4, it is preferable that the procedure of the method transfers the magnetized second cells onto the first cells in a state in which the second cells are held by magnetic force, and then removes magnetic force, thereby seeding the second cells on the first cells. This method makes it possible to transfer the second cells to a targeted position (predetermined position on the first cells) easily and reliably. In particular, in case where the magnetized second cells are in a form of monolayered or multilayered fragile sheet, this method makes it easy to handle such second cells and makes it possible to transfer such second cells while easily maintaining the layer form or the planar shape. Consequently, transferring or seeding by magnetic force is an effective method capable of effectively avoiding damage to the second cells and obtaining a desirable multilayered form. Since the second cells can be seeded on the predetermined position on the first cells, this method can seed the second cells without disturbing the state in which the first cells are held, accumulated, or shapeed of a layer. Therefore, this method can be a transferring and seeding method that is capable of avoiding damage to the first cells. Transferring by magnetic force can use a suspending support film, which can be used in a recovering step mentioned below. The cultured tissue of the present invention can be prepared by culturing the magnetized first, second and third cells in parallel so as to form monolayered cell sheet or multilayered cell sheet and then by multilayering the formed cell sheets while producing magnetic induction so as to sequentially attract the cell sheets to a magnetic source from the upper part of the container by magnetic force.

### (Magnetic induction step)

In the magnetic induction step of the present invention, magnetic force may be operated to magnetically induce magnetized cells to a desirable position. For allowing magnetic force to act so that cells are attracted to the culture surface, magnetic induction by placing a magnet on the side opposite to the cell culture side via the culture surface (the side facing the cells with the culture surface sandwiched therebetween) can be employed. When the surface of a plate-shaped culture substrate is used as a culture surface, a magnet can be placed on the rear surface side. When the outer surface (outer circumferential surface) of a tubular-shaped culture substrate is used as a culture surface, a magnet can be placed inside the tubular-shaped culture substrate.

For example, in the case of attracting magnetized cells to a culture surface by a magnet set at the opposite side of the culture surface of the culture substrate, magnetic force can be appropriately determined based on kinds of magnetic particles, an amount of magnetic particles taken up by cells, material quality of the culture surface of the culture substrate, the thickness of the culture surface, and the like. In this magnetic induction step, it is also possible to transfer cells to a predetermined position by magnetic force concomitantly with attracting and disposing cells to a predetermined position by magnetic force.

This magnetic induction step can attract (induce) and dispose the second cells onto a desirable position with respect to the first cells by magnetic force. Consequently, it is possible to construct a desirable multilayered structure, and a desirable three-dimensional structure.

The position to which the second cells are attracted may be any places in which at least a part of the second cells overlaps a group of the first cells (cell accumulation or a cell sheet). At the overlapping site, the first cells and second cells are brought into contact with each other and cultured. Thereby, both cells are bound to each other and can be multilayered. Consequently, the second cells are not necessarily multilayered on the entire first cell group but may be partially multilayered thereon.

In the above-mentioned preparation step, in case where the first cells are prepared in a form of cell accumulation or cell sheet, it is possible to carry out a magnetic induction step that attracts the first cells, which was previously magnetized by allowing them to hold magnetic particles, onto the culture surface of the culture substrate by magnetic force. In other words, the procedures can magnetize the first cells in a similar way to magnetization of the second cells and allows the magnetized first cells to be held, accumulated or cultured on the culture surface of the culture substrate. By holding, accumulating or forming a sheet of magnetized first cells by carrying out such a magnetic induction step, monolayered or multilayered first cells can be prepared in a simple way for a short time. When a culture substrate having a cell non-adhesive culture surface is used, the cultured tissue can be released from the culture substrate easily by releasing magnetic force after cultured tissue is constructed.

### (Culture step)

A culture step cultures the first cells and second cells in a cell arrangement obtained in the magnetic induction step. Thus, an extracellular matrix is produced by the first and second cells, and the cells adhere to each other strongly. In order to maintain the arrangement of cells (magnetic induction state) obtained by the magnetic induction step, it is preferable to magnetically induce so that magnetic force acts continuously also in the culture step. In this culture step, when the first cells are cultured to an extent that they are inhibited from proliferating by contact inhibition, the second cells are proliferated. when the first cells are not cultured to such an extent, or first cells are only held or accumulated and they can be proliferated or an extracellular matrix has not been formed yet, in the cell culture step, both the first cells and the second cells may be substantially proliferated. The first cells proliferate in a form of a layer prepared on the culture surface and the second cells proliferate so that they are multilayered on the site attracted (induced) to the first cell layer by magnetic force.

In this culture step, the culture conditions of the first and second cells, proliferation state to be obtained by the culture, and the like, are not particularly limited, and the first and second cells may be cultured until a targeted state can be obtained. The first cells and the second cells may form a monolayer or multilayer, respectively.

According to this culture method, since cells to be multilayered can be disposed and adhere to an arbitrary position by magnetic force, cultured tissue having a desirable three-dimensional shape can be obtained. Furthermore, by forming a desirable state of multilayer of the same type or different types of cells, cultured tissue approximating to the original tissue form can be formed easily, and since an environment that is suitable for cells to function can be constructed, excellent cultured tissue can be obtained.

In addition, in this culture method, as previously mentioned, third cells or any more cells may be multilayered. This culture method can produce magnetic induction to attract the third cells or any more cells to a predetermined position on the cells of the lower position (which means, for example, the first and/or the second cells with respect to the third cells), so as to dispose the third cells or any more cells in a position that is in the vicinity of or brought into contact with the cells of the lower position, and allow the cells to adhere to each other. Therefore, the third cells may be brought into contact with and adhere to the first cells. It is preferable that the third cells and any more cells have cell adhesion-dependent property, similarly to the first and second cells. The third cells and any more cells may be in a dispersion form, monolayered or multilayered form (including a sheet form). Furthermore, the third cells and any more cells are magnetized with magnetic particles, etc. and cultured in a state in which they are disposed by the use of magnetic induction by magnetic force.

In this culture step, the kinds of liquid media can be appropriately selected depending upon types of cells to be cultured. For example, well-known DMEM, α-MEM, M199, and the like, may be selected as a medium. Furthermore, additive factor such as growth factor represented by EGF or FGF may be appropriately added.

### (Recovery step)

The cell culture method of the present invention may include a recovering step that recovers cells, which reached a predetermined state, by magnetic force. In this recovering step, the cells may be recovered by putting a suspending support film in a culture substrate, allowing the cells that reached a predetermined state to be attracted to the support film by magnetic force, and then lifting the suspending support film. Herein, the suspending support film is not particularly limited, but any one may be employed as long as it can suspend the cells that reached a predetermined state substantially as it is. Examples of such a suspending support film may include knit fabric, woven fabric, non-woven fabric, paper, resin sheet, and the like. For example, in addition to sterile gauze, sterile Japanese paper, sterile filter paper and sterile non-woven fabric, a hydrophilic film (including a film the surface of which was treated to have hydrophilic property) such as a PVDF film (polyvinylidene fluoride film), a PTFE film (polytetrafluoroethylene film), etc., a sheet-like material of macromolecular materials with flexibility such as silicon rubber, a biodegradable polymer such as polyglycolic acid, polylactic acid, etc., and hydrogel such as agar medium, collagen gel, gelatin gel, fibrin gel, alginate gel, poly-N-isopyl-acrylamide gel, etc., and the like. As the magnetic force, magnetic force of an electromagnet capable of controlling the magnetization and demagnetization by being energized and de-energized may be used. This is convenient because not only an operation of recovering cultured tissue that reached a predetermined state can be easily automated but also operations of transferring and packaging cells can be easily automated. As mentioned above, prior to recovering cultured tissue, cultured tissue can be released from the culture surface by removing or reducing magnetic force applied to the culture surface. The cultured tissue can be recovered by grasping the cultured tissue suspended to a suspending support film with tweezers, or sucking with a pipette having a large hole diameter.

In the cell culture method of the present invention, a culture step may be omitted. In the magnetic induction step, by attracting the second cells onto a predetermined position on the first cells by magnetic force, even if the first cells and second cells are not cultured, it is possible to obtain multilayered tissue in which the first cells and second cells are substantially integrated in a state in which they are multilayered. This method can provide multilayered tissue in a simple way. For example, in case where the second cells are supplied (seeded) to the first cells in a form of a monolayered or multilayered sheet, the second cells can be integrated with the first cells easily by producing magnetic induction so as to attract the second cells to a predetermined position by magnetic force. This is much more for the case in which the second cells are supplied in a form of two sheets or more.

In case where the first cells and second cells adhere to each other only by a magnetic induction step without carrying out a culture step, the adhesion strength depends upon kinds of cells or the seeding form of the second cells. In case where the second cells are in a form of sheet, etc., the adhesion strength is relatively high and the strength of the entire multilayer tissue is also high, the cultured cells can be released and recovered from the culture surface by removing magnetic force from the culture surface.

On the other hand, in case where the cultured cells have a low adhesion strength between the first cells and second cells multilayered only by the magnetic induction step, or have low entire strength, cultured cells can be recovered after carrying out an integrating step that binds and integrates the first cells and second cells to each other with a binding material in a cell arrangement obtained in the magnetic induction step. By integrating the first cells and the second cells with each other by using a binding material, the strength of the cultured cells can be improved and the cultured cells can be released and recovered from the culture surface without destroying the integrated structure. As the binding material, bio-compatible gelling materials can be preferably used and, for example, collagen, gelatin, agarose, fibrin, alginic acid, polyvinylalcohol hydrogel can be used singly or in a form of a mixture of two or more thereof. When the gelling material is used, the binding and integration of cells are achieved by gelation of the gelling material. By using the gelling material, cells to be bound can be embedded easily, the integration of cells can be improved and the releasing and recovering operation can be facilitated. To integrate the cells in the arrangement of cells obtained in the magnetic induction step, gelling is carried out in a state in which the action of magnetic force used in the magnetic induction step is maintained as it is, thus allowing the binding force by the binding materials to be acted. By carrying out the integration with the action of magnetic force maintained, the state in which the cells are induced and fixed can be maintained. Therefore, the first cells and second cells can be fixed while reliably positioning the first cells and second cells at a predetermined position. After integration, after removing the magnetic force, that is, removing a magnet, the cultured cells can be recovered from the culture surface.

When the above-mentioned integration step is also applied to cultured cells obtained by carrying out the culture step, it is possible to obtain cultured cells that have high strength and can be handled easily as well as it is possible to recover the cultured cells from the culture surface easily. Furthermore, the above-mentioned integration step can facilitate recovering cultured cells from a culture surface in the case where the culture step is carried out after the magnetic induction step, so that the adhesion of cells to the culture surface is enhanced, or in the case where the culture step is carried out on a surface that is not cell non-adhesive culture surface.

The cultured tissue obtained by this culture method has a multilayered product of cells and has magnetized cells in at least a part of the multilayered product. It is preferable that almost all the cells of this cultured tissue are magnetized. Furthermore, this cultured tissue can be composed of a combination of cells that can be applied to the cell culture method of the present invention. This cultured tissue contains magnetized cells, so that the cultured tissue can be transferred or fixed, etc. by magnetic force. Additional three-dimensional structure can be constructed based on the magnetic property of the cultured tissue. Therefore, it is possible to provide easy-to-use cultured tissue. The cultured tissue of the present invention can provide tubular-shaped cultured tissue, and cultured tissue forming a part or entire of spherical surface in addition to substantially sheet-like cultured tissue. Examples of cultured tissue forming a part of spherical surface include dish-shaped tissue and a circular dish-shaped tissue such as cornea tissue. Examples of the cultured tissue forming an entire spherical surface include cultured tissue formed on a surface of a spherical culture substrate that can be applied to a microcarrier culture method, etc.

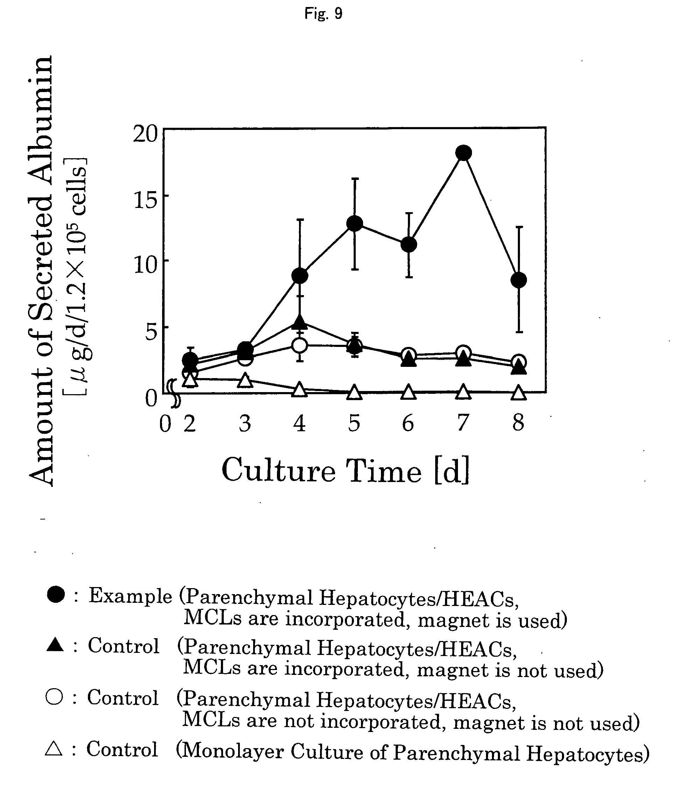

The formed cultured tissue can be used for prosthetic material for transplantation for mending or substituting a defective site or a lesion site of a patient, tissue equivalent for experiment used for screening or toxicity test of pharmaceutical preparation, cosmetics, and the like. Since the cultured tissue can be applied for a microcarrier culture technology for extracting and recovering cytokine or protein produced by cells, as compared with a conventional method for culturing a single kind of cells and extracting thereof, it can be expected that the amount of production of cells is increased by increasing the number of cells capable of being held by each microcarrier (multilayering), or the amount of production of cells is increased by multilayering two different kinds of cells on the microcarrier, and that cytokine or protein, which cannot be produced by a single kind of cells, is extracted. This can be anticipated from the following Fig. 9, which shows that cultured tissue obtained by multilayering parencymal hepatocytes and vascular endothelial cells secretes a larger amount of albumin as compared with cultured tissue formed of only parencymal hepatocytes.

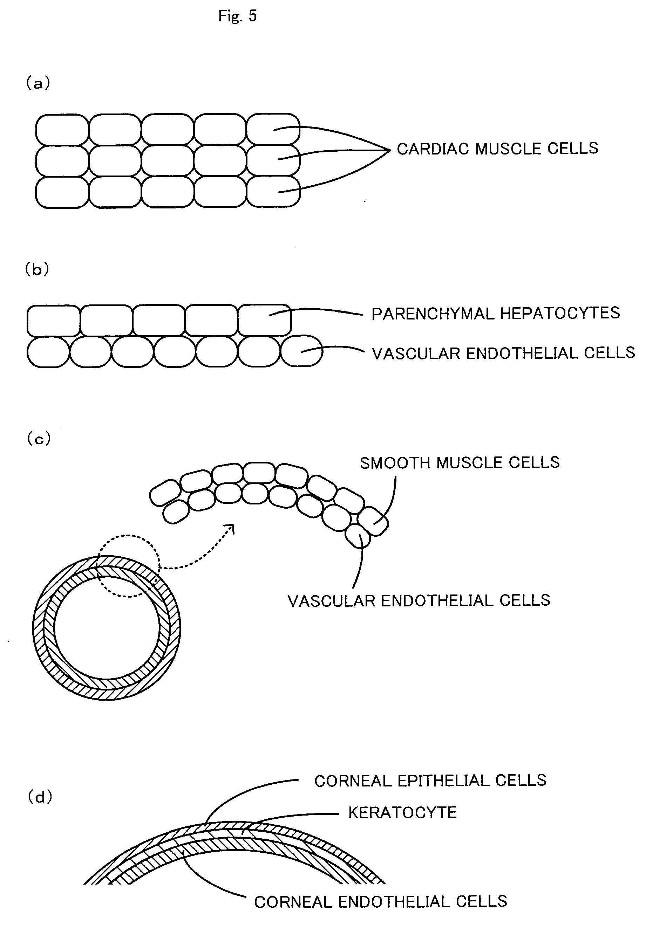

Fig. 5 shows examples of cross-sectional structures of various three-dimensional cultured tissues obtained by the cell culture method of the present invention. Fig. 5(a) shows a substantially sheet-like cultured tissue obtained by multilayering the same type of cells (this figure shows cardiac muscle cells). Fig. 5(b) shows a substantially sheet-like cultured tissue obtained by multilayering different types of cells (this figure shows vascular endothelial cells and hepatocytes). Fig. 5(c) shows a substantially tubular-shaped cultured tissue obtained by multilayering smooth muscle cells on the outer circumference of the vascular endothelial cells that were prepared in a tubular shape. The cultured tissue shown in Fig. 5(c) can be also obtained by using the inner surface of the tubular-shaped culture substrate as a culture surface, preparing the smooth muscle cells on this culture surface, and disposing the vascular endothelial cells with respect to the smooth muscle cells so as to form a multilayer structure. Furthermore, the outer circumference of the smooth muscle cells may be provided with fibroblasts. Fig. 5(d) shows a substantially circular dish-shaped cultured tissue obtained by multilayering keratocyte and corneal epithelial cells in this order on the upper surface of the circular dish-shaped product of corneal endothelial cells. The cultured tissue shown in Fig. 5(d) can be also obtained by using the inner surface of the circular dish-shaped culture substrate as a culture surface, preparing corneal epithelial cells on this culture surface, disposing keratocytes with respect to this corneal epithelial cells, and disposing corneal endothelial cells with respect to the keratocytes so as to form a multilayer structure.

### [Example 1]

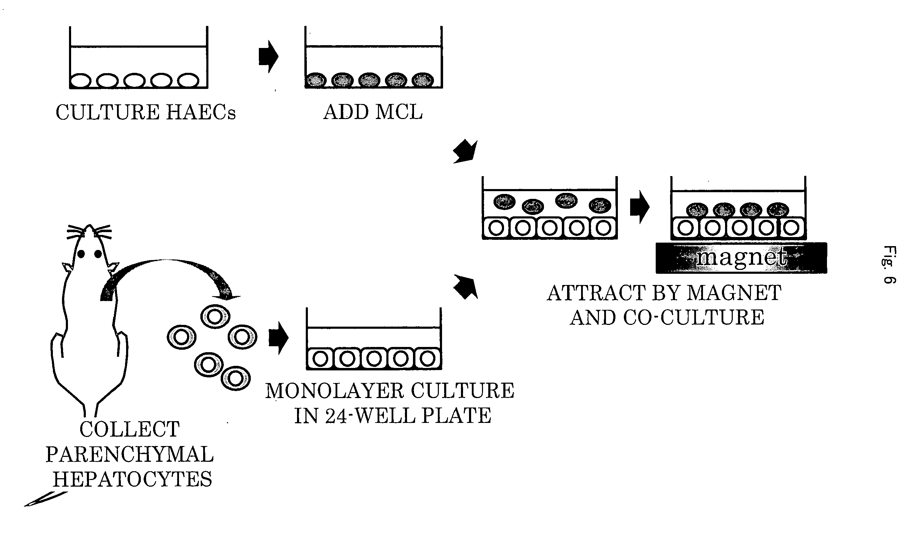

Hereinafter, the present invention will be specifically described by way of Examples. However, it is not intended that the present invention is limited to these Examples. In the following Example 1, a case in which cultured liver tissue was constructed by using parencymal hepatocytes and vascular endothelial cells will be described. Fig. 6 schematically shows this Example.

### (1) Harvest of rat parencymal hepatocytes

Rat parencymal hepatocytes were harvested from 7-9 week-old Sprague-Dawley rat by an in situ collagenase perfusion method. The collagenase perfusion method is generally described in Toshikazu Nakamura, Experimental Method for Primary Culture of Liver, Center for Academic Publications Japan, 1987; 5-17 and Seglen PO. Preparation of isolated rat liver cells, Meth. Cell. Biol 1976; 13: 29-83. In this Example, rat parencymal hepatocytes were harvested by the following method.

### (Collagenase Perfusion Method)

The procedure of the method firstly anesthetized a rat by inhalation of diethyl ether and intraperitoneal injection of Nembutal, then soaked the rat in 0.05% Osvan solution (37°C) for disinfection, fixed the rat on a dissection table, and incised the skin and abdominal muscle in this order so as to expose the abdominal cavity. The procedure drew the intestines to the right side seen from an operator to sufficiently expose the portal vein and passed a suture under the portal vein to form a loop. Cannulation was carried out by inserting a cannula with an indwelling needle into the portal vein from the downstream of the suture, then pulling out the indwelling needle, inserting the cannula that is an outer casing in the direction of the liver and fixing with the suture. Since blood flew through the cannula, exsanguinations was carried out for 30 seconds.

The procedure allowed a preperfusion solution (37°C) to flow through the cannula at 30 ml/min, and cut the inferior vena cava under the liver after about one minute when the liver swelled. When the inferior vena cava was cut, blood flew out at one time and the blood was exsanguinated. The procedures opened the thorax part to expose the heart, then clamped the cut inferior vena cava with forceps, formed a loop of a suture on the inferior vena cava located under the diaphragm and entering the right atrium, and allowed the cannula to pass through the inferior vena cava provided with a loop from the right atrium by using a dwelling needle. The procedure further inserted the cannula in the direction of the liver and fixed it by the suture. The preperfusion solution was allowed to flow out of the cannula that had been inserted into the right atrium. The preperfusion solution was continued to flow until blood was completely removed from the flow-out preperfusion solution (for about 25 minutes).

The preperfusion solution was replaced by a collagenase solution and the collagenase solution was allowed to flow at 30 ml/min for 5 minutes. Thereafter, the flow rate was reduced. The treatment with collagenase gradually digested the liver, so that the hepatic lobule came to the surface and the collagenase solution exuded from the surface. After about 10 minutes of the treatment with collagenase, the perfusion was stopped. Then, the procedure cut the connection parts between the liver and the diaphragm and blood vessel, cut the connection parts between the liver and the other tissues with scissors while picking the liver with tweezers, and transferred the liver into a 10 mm dish filled with a sufficient amount of a MEM solution containing 5% FCS. The liver was cut into pieces with scissors and the dish was shaken so as to exude cells. After cells were sufficiently dispersed, a cell suspension was filtrated by passing it through three sheets of gauzes superimposed on a 100 mm metallic mesh. The obtained cell suspension was centrifuged (50×g, 1 minute) by the use of washing buffer solution four times so as to separate parencymal hepatocytes from non-parencymal hepatocytes. Cells settled in this centrifugation were parencymal hepatocytes. These settled fractions were collected as parencymal hepatocytes.

The numbers of viable cells in the obtained cells were counted by a trypan blue method for evaluating cell viability and only the cells whose viability of 80% or more were used for experiment. The cells were seeded at 6 x 10⁴cells/cm² and cultured in a parencymal hepatocyte culture medium on a type I collagen coated dish (Asahi techno glass). Compositions of the preperfusion solution, collagenase medium, washing buffer solution, and parencymal hepatocyte culture medium are shown in the following Tables 1 to 4, respectively.

**Table 1 Composition of preperfusion solution (unit: g/l)**

| | |
|---|---|
| NaCl | 8 |
| KCl | 0.1 |
| NaH₂PO₄ ·2H₂O | 0.1014 |
| Na₂HPO₄ | 0.06 |
| HEPES | 2.38 |
| Phenol red | 0.006 |
| EGTA | 0.19 |
| NaHCO₃ | 0.35 |
| Glucose | 0.9 |

| | |
|---|---|
| (Every ingredient is product of Wako Pure Chemical) | |

**Table 2 Composition of collagenase solution (pH 7.5) (unit: g/l)**

| | |
|---|---|
| NaCl | 8 |
| KCl | 0.1 |
| NaH₂PO₄ ·2H₂O | 0.1014 |
| Na₂HPO₄ | 0.06 |
| HEPES | 2.38 |
| Phenol red | 0.006 |
| CaCl₂ | 0.19 |
| NaHCO₃ | 0.35 |
| Collagenase | 0.5 |

| | |
|---|---|
| (Every ingredient is product of Wako Pure Chemical) | |

**Table 3 Composition of washing buffer solution**

| Minimal medium^{*1} containing the following ingredients at the following concentration | |
|---|---|
| FBS | 5% |
| Non-essential amino acid^{*1} | 10mM |
| Antibiotics^{*1} | |
| Streptomycin sulfate | 0.1mg/ml |
| Sodium penicillin G | 100U/ml |

| | |
|---|---|
| *1: products of Invitrogen | |

**Table 4 Composition of culture medium for culturing parencymal hepatocyte**

| Williams medium^{*2} containing the following ingredients at the following concentration | |
|---|---|
| CuSO₄ · 5H₂O^{*2} | 0.1 µM |
| Na₂SeO₃^{*2} | 25 nM |
| ZnSO₄ ·7H₂O^{*2} | 1.0 µM |
| Insulin^{*2} | 0.1 µM |
| Dexamethasone^{*3} | 1.0 µM |
| EGF^{*2} | 20 µg/l |
| Gentamicin sulfate^{*3} | 48 mg/l |
| Chloramphenicol^{*3} | 100 mg/l |

| | |
|---|---|
| *2: product of Sigma | |
| *3: product of Wako Pure Chemical | |

### (2) Harvest of human aortic endothelial cells (non-parencymal hepatocytes)

As non-parencymal hepatocytes, human aortic endothelial cells (HAECs, Sanko Junyaku) were used. First to sixth passage of subcultured cells were used. The cells were subcutured when they were 80% confluent. A quarter of the cells were seeded. For a medium, a medium for vascular endothelial cells (EGM-2, trade name of Sanko Junyaku) containing the following ingredients was used.
[Contained ingredients] Hydrocortisone, hFGF-B, VEGF, R3-IGF-1, Ascorbic acid, Heparin, FBS, hEGF, and Gentamicin/Amphotericin B

### (3) Preparation of magnetite cationic liposome (MCL)

As magnetic particles magnetite (Fe₃O₄), magnetite (Fe₃O₄ :Toda Kogyo) having a particle size of 10 nm was used. Magnetite was washed in deionized water to sufficiently remove excessive ion components, followed by ultrasonic treatment to form a magnetite dispersion solution in which magnetites were dispersed in water. As phospholipid, N-( 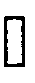 - trimethylammonio-acetyl)-didodecyl-D-gulutamate chloride (TMAG) (Sogo Pharmaceutical), Dilauroyl phosphatidylchloride (DLPC) (Sigma Chemical), and Dioleoylphosphatidylethanolamine (DOPE) (Sigma Chemical) were used, and these were dissolved in chloroform to compose them in a 1:2:2 molar ratio (TMAG : DLPC : DOPE). The procedure put this composition in a nasu flask, sucked air while rotating with the use of a rotary evaporator, and removed the solvent so as to form lipid membrane on the inner wall. To this lipid membrane, 2 ml of the previously mentioned magnetite dispersion solution (10 mg/ml) was added so as to swell the membrane while carrying out vortex agitation. The swollen membrane and magnetic particles were subjected to ultrasonic treatment (28W) for 15 minutes, and allowed to disperse in phosphate buffered saline (PBS) by adding 10-fold concentration of PBS. Furthermore, ultrasonic treatment (28W) was carried out for 15 minutes to obtain MCL.

### (4) Toxicity of MCL to human aortic endothelial cells (HAECs)

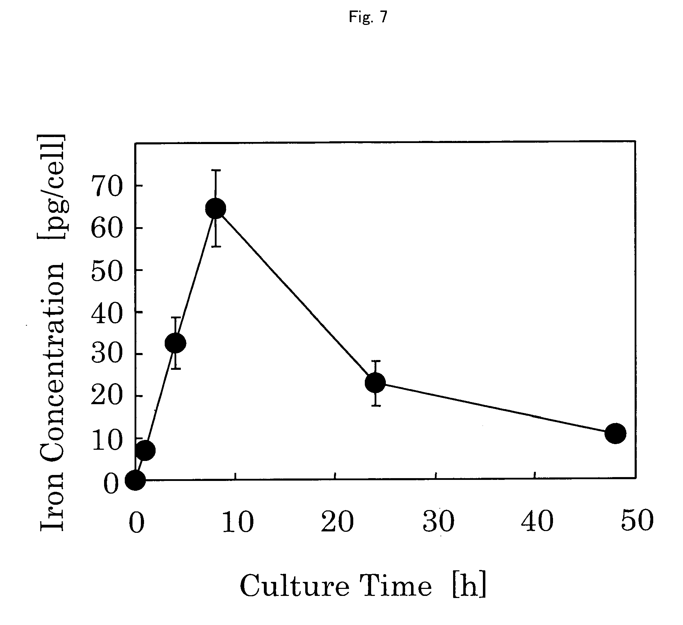

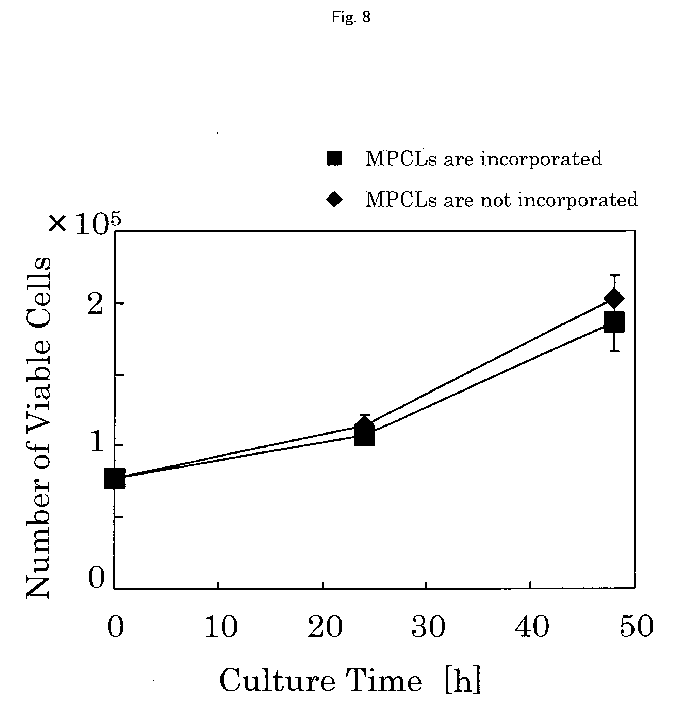

The procedure seeded HAECs on a 100 mm dish at 1.5×10⁵ cells, added MCLs onto a medium so that iron concentration became 100 pg/cell, measured the number of viable cells and intracellular iron concentration at 1, 2, 4, 8, 24 and 48 hours after the addition of MCLs. Fig. 7 shows measurement results of the intracellular iron concentration, and Fig. 8 shows measurement results of the number of viable cells. As shown in Fig. 7, when HAECs were allowed to take up MCLs at magnetite concentration of 100 pg/cell, the intracellular iron concentration became maximum (about 65 pg/cell) at 8 hours after the addition of MCLs. As shown in Fig. 8, the proliferation of cells incorporating MCLs at the maximum intracellular iron concentration was not different from that of cells which do not incorporate MCL. Thus, the toxicity of MCL to HAECs was not observed at the maximum concentration.

### (5) Culture of human aortic endothelial cells on rat parencymal hepatocytes using magnet and measurement of amount of albumin

Parencymal hepatocytes were seeded in a Williams medium on a type I collagen coated 24-well plate (Asahi Techno Glass). At two days after the start of culture, HAECs were allowed to take up MCLs, and HAECs were seeded on each well at 2×10⁵ cells/well. On the rear surface of each well, a magnet having a diameter of 1 mm (neodynium magnet, surface magnetic flux density: 0.45T) was placed and HAECs were cultured in a state in which they were attracted to the bottom of each well. As control, a co-cultured product obtained by co-culturing HAECs incorporating MCLs and parencymal hepatocytes without using a magnet, a co-cultured product obtained by co-culturing HAECs not incorporating MCLs and parencymal hepatocytes without using a magnet, and static cultured product obtained by culturing only parenchymal hepatocytes without using a magnet were used.

### (Measurement of amount of secreted albumin)

At one day after the start of co-culture, the medium was replaced by 0.5 ml of medium obtained by mixing a Williams medium and an EGM-2 medium in a ratio of 1:1. Thereafter, every 24 hours, culture supernatant was collected and cyropreserved at - 40°C and the amount of secreted albumin was measured. The measurement of albumin was carried out by measuring the amount of albumin in the collected cultured supernatant by ELISA method. ELISA method was carried out as follows.

### (ELISA method)

The procedure of the method added 100 ml/well of primary antibody (anti-rat albumin goat IgG fraction, Cappel) that had been adjusted to 10 mg/ml with PBS to a 96-well ELISA plate (Corning), incubated overnight at 4°C so as not to dry, and thereafter washed with 200 ml/well of washing solution three times. The procedure added 100 ml/well of measurement samples and incubated at 37°C for 30 minutes, then washed with 200 ml/well of washing solution three times. The procedure added 200 ml/well of secondary antibody (anti-rat albumin peroxidase-bound rabbit IgG fraction, Cappel) that had been adjusted with a washing solution, incubated for 1 hour at room temperature, washed with 200 ml/well of washing solution four times and added 200 ml/ml of substrate solution. This solution was incubated for 10-30 minutes at room temperature and 50 ml/ml of reaction stopping solution was added to the solution. The absorbance was measured by the use of spectrophotometer at the wavelength of 490 nm (control wavelength 655 nm) and the amount of albumin in the supernatant was measured. Fig. 9 shows the results.

Fig. 9 shows that the amount of secreted albumin was maximum in case where HAECs incorporating MCLs were seeded on parencymal hepatocytes and co-cultured by using a magnet (shown by black circle in Fig. 9). It was confirmed that albumin was secreted even 8 days after the start of culture.

On the contrary, in the control culturing only parencymal hepatocytes (shown by white triangle in Fig. 9) showed the albumin secretional capacity of the same level as that in the co-culture systems at the beginning of culturing, but then it rapidly reduced and at 7 days after the start of culturing, albumin could hardly detected. When a magnet is not used in the co-culture system incorporating MCLs (shown by black triangle in Fig. 9) and when HAECs not incorporating MCL were used (shown by white circle by Fig. 9), it was observed that the adhesion of HAECs to parenchymal hepatocytes accompanied by spontaneous sedimentation slightly improved and extended the amount of secreted albumin. Furthermore, there was no difference in the effect of the supersecretion of albumin between these two cases. However, both cases showed fewer amount of secreted albumin as compared with the co-culture group using a magnet.

The procedure seeded HAECs incorporating MCLs on parencymal hepatocytes seeded on a Petri perm plate and started culturing by using a magnet (diameter: 1 mm, surface flux density: 0.45T) and observed the states of cells two days after the start of the culture. It was confirmed that HAECs adhered to the parencymal hepatocytes, HAECs were present on the region in which parencymal hepatocytes were present, and HAECs were not present on the region in which parencymal hepatocytes were not present.

### (6) Culture of human aortic endothelial cell on rat parencymal hepatocytes using a magnet and observation by tissue staining

The procedure seeded parencymal hepatocytes on a Petri perm plate coated with collagen. Two days after the start of culture, the procedure allowed HAECs to take up MCL, seeded the HAECs on parencymal hepatocytes, placed a magnet having a diameter of 3 mm (neodymium magnet, surface flux density: 4000 T) under the Petri perm plate, shook it with a universal shaker for 10 minutes, and then carried out static culture. Thereafter, culture was carried out for two days with a magnet placed. At two days after the seeding of the cells incorporating MCLs, the procedure carried out 10% formalin fixation, then formed a tissue section, stained it with hematoxylin, eosin and an anti-rat albumin antibody (anti-rat albumin rabbit IgG, Cappel) and observed the tissue cross-section.

The observation of the cross section of stained tissue showed that by using a magnet, HAECs accumulated on the magnet. The culture cross section showed a state in which HAECs incorporating MCLs are multilayered and adhered onto parenchymal hapatocytes. In addition, when albumin serving as an index of hepatic function was stained with anti-albumin antibody, the bottom layer of cells were stained but a cell layer (HAECs layer) formed on the bottom layer was hardly stained with albumin. Furthermore, HAECs were hardly observed on the culture cross-section of a place in which a magnet was not placed. These results showed that when the culture was carried out in a state in which a magnet was placed and HAECs were disposed on parencymal hepatocytes by magnetic force of the magnet and were cultured, the HAECs could be multilayered on parencymal hepatocytes and that the secretion of albumin by parencymal hepatocytes was increased by multilayering non-parencymal hepatocyte HAECs on parencymal hepatocytes.

### [Example 2]

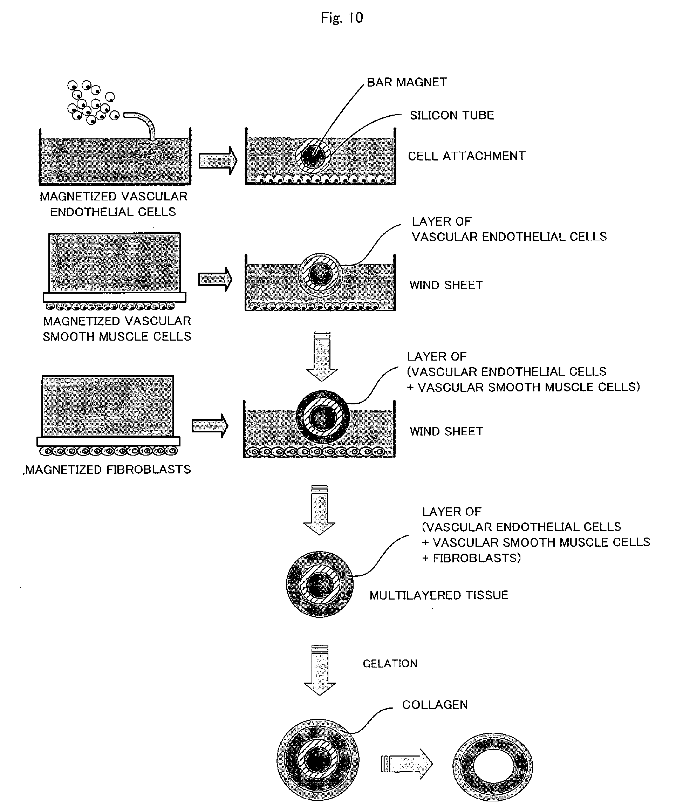

In the below-mentioned Example 2, a case in which cultured vascular tissue was constructed by using fibroblasts, vascular smooth muscle cells and vascular endothelial cells will be described. This Example constructed vascular tissue by using vascular endothelial cells as first cells, vascular smooth muscle cells as second cells, and fibroblasts as third cells. Fig. 10 schematically shows this Example.
(1) Vascular endothelial cell
   As vascular endothelial cells, human aortic endothelial cells (HAECs, Sanko Junyaku) were used. Second to fourth passage of subcultured cells were used. The cells were subcultured when they were 80% confluent. A quarter of the cells were seeded. For a medium, a medium for vascular endothelial cells (EGM-2, Sanko Junyaku) containing 0.04% hydrocortisone, 0.4% hFGF-B, 0.1% VEGF, 0.1% R3-IGF-1, 0.1% ascorbic acid, 0.1% heparin, 2% FBS, 0.1% hEGF, and 0.1% gentamicin / amphotericin was used.
(2) Vascular smooth muscle cells
   As vascular smooth muscle cells, human aortic smooth muscle cells (SMCs, Sanko Junyaku) were used. Second to fourth passage of subcultured cells were used. The cells were subcutured when they were 80% confluent. One-eighth of the cells were seeded. For a medium, a medium for smooth muscle cells (SmGM-2, Sanko Junyaku) containing 0.1% hEGF, 0.1% insulin, 0.2% hFGF-B, 5% FBS, and 0.1% GA-1000 was used.
(3) Fibroblasts
   For fibroblasts, NIH/3T3 cells derived from mouse were used. For a medium, cMEM supplemented with 10% FBS, 100 U/ml penicillin/0.1 mg/ml streptomycin and 1% non-essential amino acid was used.
(4) Culture substrate and magnet
   As a culture substrate, a general culture dish having a cell adhesion property and a 24-well plate ultra-low-attachment dish (Coaster) were used. The general culture dish was used for subculture of the respective cells (vascular endothelial cells, vascular smooth muscle cells, and fibroblasts) and taking up of MLCs. On the other hand, the ultra-low-attachment dish was used for forming a vascular smooth muscle cell sheet (SMC sheet) and a fibroblasts sheet (3T3 sheet). Electrical neutral hydrophilic gel was bound on the surface of this ultra-low-attachment dish. Therefore, protein or other biomolecules adsorbed to the surface by hydrophobic interaction or electric interaction cannot be adsorbed to the surface of this dish. In other words, cell adhesion factors cannot be adsorbed to the dish, thus inhibiting cell adhesion accompanied therewith. Culturing of these cell sheets used a columnar-shaped neodymium magnet (diameter: 30 mm, surface flux density: 0.45T) (As One). The procedure of forming vascular tissue used a bar-shaped neodymium magnet (diameter: 3 mm, length: 10 mm, surface flux density: 0.28T). Both ends of the longitudinal direction of this neodymium magnet are magnetized to the S pole and N pole, respectively.
(5) Formation of vascular smooth muscle cell sheet (SMC sheet) and fibroblast sheet (3T3 sheet)
   The procedure counted the number of cells on a culture dish in a state in which respective cells (vascular smooth muscle cells and fibroblasts) became 80% confluent, then added MCLs to the culture dish at the concentration of 100 pg/cell and incubated for 4 hours. Similar to the subculture operation, this procedure carried out a treatment with a trypsin/EDTA solution, collected the cells, and centrifugation was carried out at 1000 rpm for 5 minutes. Then, the cells were suspended in each of the above-mentioned media. The procedure seeded cells to the 24-well plate ultra-low-attachment dish at 2x10⁶ cells/well, which was 5 times number of cells corresponding to the confluent, and then placed a neodymium magnet (outer diameter: 30 mm) under the well so that the well was located at the center of the neodymium magnet. At one day, the cells were recovered as a cell sheet. The procedure removed the magnet and then tapped the edge of the dish so as to allow the cell sheet to float up. The cell sheet was recovered with a medium by using a pipette whose tip has increased hole-diameter.
(6) Preparation of vascular endothelial cells
   The procedure counted the number of cells on a culture dish in a state in which vascular endothelial cells became 80% confluent, then added MCLs to the culture dish at the concentration of 200 pg/cell and incubated for 4 hours to magnetize the cells. Similar to the subculture operation, this procedure carried out a treatment with a trypsin/EDTA solution, collected the cells, and centrifugation was carried out at 1000 rpm for 5 minutes. Then, the cells were suspended in a medium and the cell concentration was adjusted to 4×10⁶cells/ml. Vascular endothelial cells (HAECs) were used for the following experiment in a form of suspension cells without forming a cell sheet.
(7) Formation of multilayered structure by magnetic induction
   The procedure placed the above-mentioned bar magnet having a diameter of 3 mm and a length of 10 mm in a silicon tube having an inner diameter of 3 mm, an outer diameter of 5 mm and a length of 20 mm. The procedure wound vascular endothelial cells (HAECs), a vascular smooth muscle cell sheet (SMC sheet) and fibroblast sheet (3T3 sheet) around the outside of the silicon tube in this order. As shown in Fig. 10, firstly, the procedure dropped 500 µl of suspension of vascular endothelial cells (concentration: 4x10⁶cells/ml) into a culture dish having a diameter pf 10cm and formed a pool of water having a diameter of about 15 mm. The procedure rolled the silicon tube including a magnet horizontally and allowed the cells to adhere to the silicon tube uniformly. Furthermore, the procedure spread the formed SMC sheet on another culture dish having a diameter of 10 cm and rolled the silicon tube, on the surface of which endothelial cells are attracted and which includes a magnet, on the spread SMC sheet so as to wind the SMC sheet around the silicon tube. The procedure wound 3 to 4 sheets of SMC sheets in order to completely cover the whole surface. The procedure wound 3 to 4 sheets of 3T3 sheets thereon by the similar procedure of the SMC sheets. In this way, by attracting these cells so that the cells were multilayered, vascular smooth muscle cells adhered to vascular endothelial cells and fibroblasts adhered to vascular smooth muscle cells and thus a multilayer structured vascular tissue, that is, cultured cells (tissue structure) could be obtained.
(8) Integration by collagen gel
   For gelling material, collagen (Cellmatrix Type 1-A (Nitta Gelatin)) was used. The procedure mixed Cellmatrix Type 1-A, a 10-fold concentrated solution of DMEM (Dulbecco's modified Eagle's medium) that did not contain NaHCO₃ and was supplemented with 10% FBS and penicillin/streptomycin, and a buffer solution for reconstructing collagen gel in the ratio of 8 : 1 : 1 on ice to prepare a collagen gel solution. The formed cultured cells were placed into a cylinder (length: 50 mm) cutting two places of 2.5 ml syringe (TERUMO) having a somewhat larger inner diameter (9 mm) than the outer diameter of the above-mentioned silicon tube, and further the gel solution was poured. Incubation was carried out for 30 minutes for gelation.
(9) Recovery of cultured cells

After gelation, the cultured cells together with the collagen gel were taken out from the cylinder, a magnet was pulled out from the silicon tube and the silicon tube was pulled out from the cultured cells. Thus, a tubular-shaped cultured cells fixed by the collagen gel could be obtained. The cross-section of the obtained cultured cells was observed with a microscope and a cross-sectional structure in which vascular endothelial cells, vascular smooth muscle cells and fibroblasts were integrated in this order from the inner layer was confirmed.

Note here that Example 1 uses parencymal hepatocytes as first cells and aortic endothelial cells as second cells, and Example 2 uses vascular endothelial cells as first cells, vascular smooth muscle cells as second cells and fibroblasts as third cells. Both Examples show examples using different kinds of cells. With respect to the origin of cells, Example 1 uses cells derived from rat and cells derived from human, and Example 2 uses cells derived from human and cells derived from mouse. Both Examples show examples of using xenogenic cells. In this way, these Examples described the present invention by using xenogenic cells. However, the present invention may form multilayered cells composed of allogenic or autogenous cells in accordance with the purposes.

## Claims

1. A cell culture method, comprising:
a preparation step that prepares first cells on a culture surface of a culture substrate, the first cells being adhesion-dependent cells and monolayered or multilayered cells;
a seeding step that seeds second cells on the first cells, the second cells being adhesion-dependent cells and magnetized cells;
a magnetic induction step that attracts the second cells to a predetermined position on the first cells by magnetic force; and
a culture step that cultures the first cells and the second cells in a cell arrangement obtained by the magnetic induction step.

2. The cell culture method according to claim 1, wherein the second cells are seeded in a state in which they are monolayered or multilayered.

3. The cell culture method according to claim 1 or 2, wherein the preparation step comprises a culture step that cultures the first cells until a monolayered or multilayered cell sheet is formed.

4. The cell culture method according to any of claims 1 to 3,
wherein the preparation step comprises a magnetic induction step that attracts the first cells, which are magnetized, to the culture surface of the culture substrate by magnetic force.

5. The cell culture method according to any of claims 1 to 4,
wherein the culture substrate for culturing the first cells and the second cells has any of a plate shape, a tubular shape, an envelope shape having a hollow, a columnar shape, a dish shape and a spherical shape.

6. The cell culture method according to any of claims 1 to 5,
wherein the culture surface of the culture substrate is cell non-adhesive.

7. The cell culture method according to any of claims 1 to 6,
wherein a combination of the first cells and the second cells is a combination of different types of cells.

8. The cell culture method according to any of claims 1 to 7,
wherein the combination of the first cells and the second cells is any of combinations of parencymal hepatocytes and vascular endothelial cells, fibroblasts and epithelial cells, smooth muscle cells and vascular endothelial cells, keratocytes and corneal epithelial cells, and keratocytes and corneal endothelial cells.

9. The cell culture method according to any of claims 1 to 8, further comprising a releasing step that releases cultured cells obtained in the culture step from the culture substrate.

10. The cell culture method according to any of claims 1 to 9, further comprising a recovering step of the cultured cells.

11. A cell culture method, comprising:
a preparation step that prepares first cells on a culture surface of a culture substrate, the first cells being adhesion-dependent cells and monolayered or multilayered cells;
a seeding step that seeds second cells on the first cells, the second cells being adhesion-dependent cells and magnetized cells; and
a magnetic induction step that attracts the second cells to a predetermined position on the first cells by magnetic force.

12. The cell culture method according to claim 11, wherein the second cells are seeded in a state in which they are monolayered or multilayered.

13. The cell culture method according to claim 11 or 12, further comprising an integration step that integrates the first cells and the second cells with a binding material in a cell arrangement obtained in the magnetic induction step.

14. The cell culture method according to claim 13, wherein the binding material is a gelling material, and the first cells and the second cells are integrated by gelation of the gelling material.

15. Cultured tissue having a three-dimensional shape, which is obtained by the cell culture method according to any of claims 1 to 14.

16. Cultured tissue having a multilayered product of cells,
wherein at least a part of the multilayered product of cells includes magnetized cells.

17. The cultured tissue according to claim 16, wherein different types of cells that are multilayered.

18. The cultured tissue according to claim 16 or 17, wherein the multilayered product of cells comprises different types of cells that are multilayered, and the different types of cells are any of combinations of parencymal hepatocytes and vascular endothelial cells, fibroblasts and epithelial cells, smooth muscle cells and vascular endothelial cells, keratocytes and corneal epithelial cells, and keratocytes and corneal endothelial cells.

19. The cultured tissue according to any of claims 16 to 18, which is substantially sheet-like cultured tissue.

20. The cultured tissue according to any of claims 16 to 18, which is tubular-shaped cultured tissue.

21. The cultured tissue according to any of claims 16 to 18, which is a shape of a part or entire of spherical surface.
